# EUROPEAN PATENT APPLICATION

(11) **EP 1 994 957 A1**
(43) Date of publication of application: **26.11.2008**
(21) Application number: 07737486.6
(22) Date of filing: 28.02.2007
(51) Int. Cl.: A61N 1/30

(54) **IONTOPHORESIS DEVICE AND METHOD OF MANUFACTURING THE SAME**

(30) Priority: 01.03.2006 JP 2006054516
(71) Applicant: TTI ELLEBEAU, INC., Tokyo 140-0002 (JP)
(72) Inventor: HAYAKAWA, Yoshitsune, Tokyo 150-0022 (JP); MITSUGUCHI, Kazuma, Tokyo 150-0022 (JP); KOSHIBA, Nobuharu, Tokyo 150-0022 (JP)
(74) Representative: Schäfer, Matthias W.
(86) International application number: PCT/JP2007/053730
(87) International publication number: WO 2007/099985

(57) **Abstract**

Provided is an iontophoresis device including an electrode assembly which holds a drug solution containing a salt of a first drug with an acid dissolved therein and has a polarizable electrode to be in contact with the drug solution, in which the polarizable electrode to which a second drug of the same kind as that of the first drug is adsorbed. The iontophoresis device preferably employs: the first step of adsorbing the second drug to the polarizable electrode; and the second step of bringing the drug solution into contact with the polarizable electrode, the second step being performed after the first step. As a result, the iontophoresis device is capable of suppressing a change in the pH value of the drug solution in the case where the drug solution containing the salt of the first drug with the acid dissolved is held in contact with the polarizable electrode.

## Description

### Technical Field

The present invention relates to an iontophoresis device utilizing a polarizable electrode and a method of manufacturing the device, in particular, an iontophoresis device capable of suppressing a pH change at a polarizable electrode and a method of manufacturing the device.

### Background Art

Iontophoresis involves driving a drug dissociated to positive or negative ions (drug ions) by means of a voltage to transdermally transfer the drug into an organism, and has advantages such as a reduced load to a subject and excellent controllability of the amount of the drug to be administered.

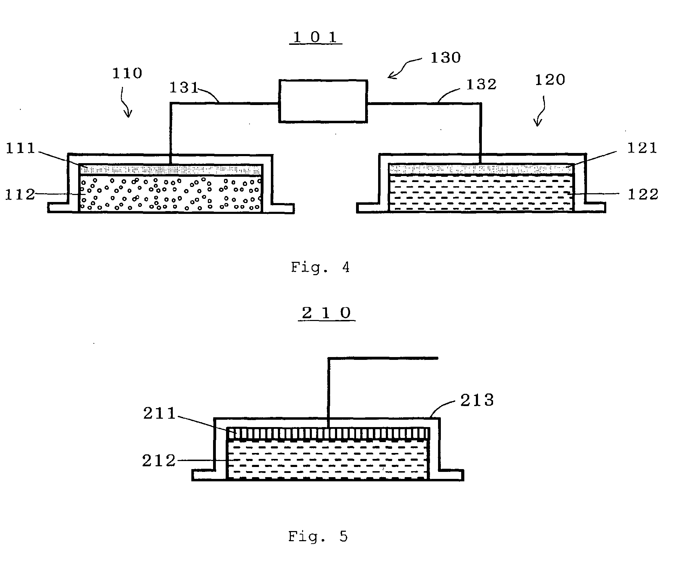

Fig. 4 is an explanatory view for showing the basic configuration of an iontophoresis device 101 as a device for performing the above iontophoresis.

As shown in the figure, the iontophoresis device 101 includes: a working electrode assembly 110 which includes an electrode 111 and a drug solution holding portion 112 for holding a drug solution containing a positive or negative drug ion; a non-working electrode assembly 120 which includes an electrode 121 and an electrolyte solution holding portion 122 for holding an electrolyte solution; and an electric power source 130 both the terminals of which are connected to the electrodes 111 and 121 through electric supply lines 131 and 132. The drug ion is administered to a living organism by applying a voltage identical in polarity to the drug ion to the electrode 111 and a voltage opposite in polarity to the drug ion to the electrode 121 in a state where the drug solution holding portion 112 and the electrolyte solution holding portion 122 are brought into contact with the skin of the living organism.

One of the problems to be solved in the iontophoresis device 101 is the occurrence of various electrode reactions upon energization in the electrode assemblies 110 and 120.

For example, when the drug ion in the drug solution is a positive ion, oxygen gas, chlorine gas, a hydrogen ion, or hypochlorous acid may be generated at the electrode 111 owing to an electrode reaction, and, depending on the kind of a drug used, the drug may cause alter the chemical reaction. Meanwhile, a hydrogen gas or a hydroxide ion may be generated at the electrode 121.

Similarly, when the drug ion in the drug solution is a negative ion, hydrogen gas or a hydroxide ion may be generated at the electrode 111 owing to an electrode reaction, and, depending on the kind of the drug used, the drug may alter the chemical reaction. Meanwhile, oxygen gas, chlorine gas, a hydrogen ion, or hypochlorous acid may be generated at the electrode 121.

When such gas as described above is generated in either of the electrode assemblies 110 and 120, energization from the electrode 111 to the drug solution and from the electrode 121 to the electrolyte solution is inhibited; when a hydrogen ion, a hydroxide ion, or hypochlorous acid is produced, such ion or acid migrates to an interface between the device and the living organism, so such ion or acid may have a harmful effect on the living organism. In addition, the occurrence of the alteration of the drug may cause the reduction or disappearance of the effect of the drug, or may lead to an unfavorable situation such as the production of a toxic substance.

A known approach to solving such problem resulting from an electrode reaction at the time of energization as described above is an approach involving the use of an active electrode such as a silver-silver chloride electrode as each electrode (for example, Patent Document 1) or an approach involving placing an electrolyte solution in which an electrolyte having a lower oxidation-reduction potential than that of water is dissolved at an interface between the electrode and the drug solution (for example, Patent Document 2).

However, the former approach makes it difficult to inhibit a reaction between the active electrode and the drug proceeding during the storage of the device, or involves the emergence of, for example, the following subsidiary problem: special measures to cope with the morphological change of the active electrode occurring upon energization are needed. As in the case of the foregoing, the latter approach involves the emergence of, for example, the following subsidiary problem: it becomes difficult to prevent the mixing of the electrolyte solution and the drug solution, and the configuration of the device must be complicated in order that one of the solutions may be separated from the mixture.

In view of the foregoing, the applicant of the present application has proposed a novel approach to solving a problem resulting from an electrode reaction at the time of energization, and has applied a patent for the approach as Japanese Patent Application No. 2005-363085 (hereinafter referred to as "prior application").

Fig. 5 is an explanatory view for showing the configuration of a working electrode assembly 210 in an iontophoresis device disclosed as one embodiment in the prior application.

As shown in the figure, the working electrode assembly 210 has: a polarizable electrode (also referred to as "electric double layer capacitor (ECDC)") 211 as an electrode having such property that energization to an electrolyte solution occurs by virtue of the formation of an electric double layer on the surface of the electrode; a drug solution holding portion 212 for holding a drug solution contacting the polarizable electrode 211, the drug solution holding portion 212 being placed on the front surface side of the polarizable electrode 211; and a container 213 for storing the polarizable electrode and the drug solution holding portion.

In the iontophoresis device including the electrode assembly 210, energization from the polarizable electrode 211 to the drug solution occurs by virtue of the formation of an electric double layer on the surface of the polarizable electrode 211, so a problem due to an electrode reaction in a conventional iontophoresis device can be alleviated or dissolved.

Further, the use of the electrode assembly 210 can solve even a problematic reaction between the electrode and the drug or a problem resulting from the morphological change of the electrode in Patent Document 1, or a problem in Patent Document 2 such as difficulty in separating the drug solution and the electrolyte solution or the complication of the configuration of the device.
Patent Document 1: US 4,744,787
Patent Document 2: JP 04-297277 A

### Disclosure of the Invention

### Problems to be solved by the Invention

However, in the course of investigation conducted by the inventors of the present invention on such iontophoresis device having a polarizable electrode contacting a drug solution as described above, the inventors have revealed the following: depending on the kind or the like of a drug to be administered to a living organism, a remarkable reduction in pH value of the drug solution occurs in some cases, and a problem such as the deterioration of a member of which the device is formed (such as the container 213 in the electrode assembly 210), or surface roughening or inflammation at the skin of the living organism occurs owing to the reduction in some cases.

The present invention has been made in view of the above problems, and an object of the present invention is to provide an iontophoresis device having a polarizable electrode contacting a drug solution, the device being capable of suppressing a reduction in pH value of the drug solution in the device, or a method of manufacturing the device.

### Means for solving the Problems

The present invention provides an iontophoresis device including an electrode assembly holding a drug solution into which a salt of a first drug and an acid is dissolved,
in which the electrode assembly has a polarizable electrode to which a second drug of the same kind as that of the first drug is adsorbed, the polarizable electrode contacting with the drug solution (Claim 1), or
a method of producing an iontophoresis device including an electrode assembly holding a drug solution into which a salt of a first drug and an acid is dissolved, the electrode assembly having a polarizable electrode contacting with the drug solution, the method including:
a first process of adsorbing a second drug of the same kind as that of the first drug to the polarizable electrode; and
a second process of bringing the drug solution into contact with the polarizable electrode, the second process being performed after the first process (Claim 9).

The inventors of the present invention have made extensive studies on an iontophoresis device having a polarizable electrode contacting a drug solution. As a result, the inventors have found that, when a drug solution in which a salt of a drug and an acid is dissolved is used as the above drug solution, an abrupt reduction in pH value of the drug solution occurs. For example, when an aqueous solution of lidocaine hydrochloride, which has a pH value of about 5 to 7, is brought into contact with a polarizable electrode composed of activated carbon fibers, the pH value reduces to about 2.5 within an extremely short time period.

According to the present invention, such remarkable reduction in pH value of a drug solution in which a salt of a drug and an acid is dissolved as described above in an iontophoresis device having a polarizable electrode contacting the drug solution can be prevented, or at least the extent to, or rate at, which the pH value reduces can be suppressed.

In addition, in the present invention, energization to the drug solution can be performed in a state where no electrode reaction is caused or an electrode reaction is reduced because energization at the polarizable electrode occurs by virtue of the formation of an electric double layer on the surface of the polarizable electrode. As a result, the generation of a gas such as an oxygen gas or a chlorine gas, the generation of an unfavorable ion such as a hydrogen ion or a hypochlorite ion, or the alteration of a drug ion due to a chemical reaction can be inhibited, or at least reduced.

The term "acid" as used in the present invention refers to a substance that increases the amount of hydrogen ions in an aqueous solution, and a typical acid is, for example, hydrochloric acid, hydrobromic acid, nitric acid, or sulfuric acid.

The first drug in the present invention is a drug to be administered to a living organism, and the drug is incorporated into the drug solution held in the electrode assembly.

Examples of the salt of the first drug and the acid in the present invention include lidocaine hydrochloride (C₁₄H₂₂N₂O· HCl) as a salt of lidocaine (C₁₄H₂₂N₂O) and hydrochloric acid, morphine hydrochloride (C₁₇H₁₉NO₃·HCl) as a salt of morphine (C₁₇H₁₉NO₃) and hydrochloric acid, and quinidine sulfate ((C₂₀H₂₄N₂O)2·H₂SO₄) as a salt of quinidine (C₂₀H₂₄N₂O₂) and sulfuric acid.

Although the salt of the first drug and the acid is generally often used for improving the solubility of the first drug in water when the first drug is not hydrophilic, the salt of the drug and the acid in the present invention does not necessarily need to be used for improving the solubility of the drug in water.

The second drug in the present invention is a drug of the same kind as that of the first drug in the drug solution. Therefore, when the first drug in the drug solution (drug to be administered to a living organism) is lidocaine, the polarizable electrode adsorbs lidocaine; when the first drug in the drug solution (drug to be administered to a living organism) is morphine, the polarizable electrode adsorbs morphine.

The polarizable electrode in the present invention is an electrode (electric double layer capacitor) having such property that energization to an electrolyte solution occurs by virtue of the formation of an electric double layer on the surface of the electrode.

A polarizable electrode containing an electric conductor having a capacitance per unit weight of 1 F/g or more, or a polarizable electrode containing an electric conductor having a specific surface area of 10 m²/g or more is preferably used as the polarizable electrode in the present invention. As a result, an iontophoresis device having the following characteristic can be realized: the amount of energization by virtue of the formation of an electric double layer on the surface of the polarizable electrode can be increased, and hence an additionally large amount of the drug ion can be administered while neither the generation of a gas or an unfavorable ion due to an electrode reaction nor the alteration of the drug ion is caused.

A metal electric conductor such as gold, silver, aluminum, or stainless steel, or a non-metal electric conductor such as activated carbon or ruthenium oxide can be used as the electric conductor to be incorporated into the polarizable electrode in the foregoing; the non-metal electric conductor is particularly preferably used as the electric conductor because the use can alleviate or dissolve the following problem: a metal ion is eluted from the polarizable electrode to migrate to a living organism. It should be noted that an effect similar to that described above can be obtained even when a metal electric conductor the surface of which is made insoluble such as alumite is used as the electric conductor of which the polarizable electrode is formed.

The polarizable electrode in the present invention can be an electrode containing activated carbon. In this case, an inexpensive, safe polarizable electrode having a high capacitance can be obtained.

Extremely ordinary activated carbon obtained by carbonizing and activating a raw material containing carbon such as a coconut shell, wood dust, coal, pitch, or coke can be used as the above activated carbon. The above activated carbon preferably has a capacitance per unit weight of 1 F/g or more, or preferably has a specific surface area of 10 m²/g or more.

Activated carbon fibers can be used as the above activated carbon to be incorporated into the polarizable electrode. In this case, the following additional effect can be obtained: the ease of handling of the polarizable electrode is improved. The activated carbon fibers to be used may be of, for example, a woven fabric shape or a non-woven fabric shape. Fibers obtained by carbonizing and activating novoloid fibers (fibers obtained by turning a phenol resin into fibers, and crosslinking the fibers to turn the molecular structure of the resin into a three-dimensional one) are particularly preferably used as the activated carbon fibers. The use can result in a polarizable electrode excellent in ease of handling, flexibility, and mechanical strength (such as a tensile strength), and having an extremely large specific surface area and a large capacitance.

It should be noted that the polarizable electrode in that case may be constituted only of the activated carbon or the activated carbon fibers, or the activated carbon or the activated carbon fibers may be blended with another component such as a binder polymer for improving the forming property, shape retentivity, or ease of handling of the polarizable electrode. Examples of the binder polymer that can be suitably used in this case include polytetrafluoroethylene and polyvinylidene fluoride. The binder polymer is blended in an amount of preferably 3 to 20 parts by weight with respect to 97 to 80 parts by weight of the activated carbon or the activated carbon fibers.

The adsorption of the drug to the polarizable electrode in the present invention may be chemical adsorption formed between a substance of which the polarizable electrode is formed and the drug (or the drug ion) as well as physical adsorption by virtue of the action of, for example, a Coulomb force or van der Waals force between the substance of which the polarizable electrode is formed and the drug (or the drug ion).

The adsorption of the second drug to the polarizable electrode in the invention according to claim 1 can be caused by treating the polarizable electrode with a solution prepared by dissolving the second drug in an organic solvent (claim 2). A first process of causing the polarizable electrode to adsorb the second drug in the invention according to claim 9 can include: a first step of impregnating the polarizable electrode with a solution prepared by dissolving the second drug in an organic solvent; and a second step of drying the polarizable electrode to vaporize the organic solvent (claim 10).

A lower alcohol such as ethanol can be preferably used as the organic solvent in that case. When a polarizable electrode containing activated carbon or activated carbon fibers is used as the polarizable electrode and lidocaine is used as the first drug, the treatment for causing the polarizable electrode to adsorb the second drug in the invention according to claim 2 or 10 can be a treatment involving impregnating the polarizable electrode with a solution of lidocaine in ethanol having a concentration of preferably 3 to 50%, more preferably 5 to 20%, or particularly preferably 8 to 12% and drying the resultant; the treatment for causing the polarizable electrode to adsorb the second drug is preferably such that the treatment involving the impregnation and the drying is repeated about two to four times. It should be noted that the concentration of the solution of lidocaine in ethanol in the foregoing is a ratio of the weight of lidocaine to the total weight of lidocaine and ethanol.

The adsorption of the second drug to the polarizable electrode in the invention according to claim 1 can be caused by treating the polarizable electrode with an aqueous solution in which a salt of the second drug and an acid is dissolved (claim 3). The first process of causing the polarizable electrode to adsorb the second drug in the invention according to claim 9 can include: a first step of impregnating the polarizable electrode with the aqueous solution in which the salt of the second drug and the acid is dissolved; a second step of washing the polarizable electrode with water, the second step being performed after the first step; and a third step of drying the polarizable electrode to vaporize moisture, the third step being performed after the second step (claim 11).

For example, when a polarizable electrode containing activated carbon or activated carbon fibers is used as the polarizable electrode and lidocaine is used as the first drug, the treatment for causing the polarizable electrode to adsorb the second drug in the invention according to claim 3 or 11 can be a treatment involving impregnating the polarizable electrode with an aqueous solution of lidocaine hydrochloride having a concentration of preferably 3 to 50%, more preferably 5 to 20%, or particularly preferably 8 to 12%, preferably washing the resultant with water, and drying the resultant; the treatment for causing the polarizable electrode to adsorb the second drug is preferably such that the treatment involving at least the impregnation and the drying is repeated about two to four times. It should be noted that the concentration of the aqueous solution of lidocaine hydrochloride in the foregoing is a ratio of the weight of lidocaine hydrochloride to the total weight of lidocaine hydrochloride and water.

The adsorption of the second drug in the invention according to claim 1 is preferably performed on the polarizable electrode that has been treated with glycerin in advance (claim 4). Alternatively, the first process in claim 9 is preferably performed after a third process of treating the polarizable electrode with glycerin (claim 12). Under such condition, particularly when the polarizable electrode is formed of activated carbon or activated carbon fibers, an affinity between the polarizable electrode and the solution prepared by dissolving the second drug in the organic solvent or the aqueous solution in which the salt of the second drug and the acid is dissolved can be improved, and hence the efficiency or ease with which the second drug adsorbs to the polarizable electrode can be improved. It should be noted that the activated carbon or the activated carbon fibers can be treated with glycerin by a known method.

The drug solution in the present invention can be held by impregnating the polarizable electrode with the drug solution (claim5). Withsuch procedure,the property with which energization from the polarizable electrode to the drug solution occurs can be improved.

Alternatively, the drug solution in the present invention can be held in the drug solution holding portion placed on the front surface side of the polarizable electrode (Claim 6). In this case, the degree of freedom in the adjustment of the amount of the drug held in the electrode assembly can be increased.

The term "drug" as used in the specification of the present invention refers to a substance, regardless of whether or not the substance is prepared, which has a certain drug effect or pharmacological action and which is applicable to an organism for purposes including: the therapy, recovery, or prevention of a disease; the promotion or maintenance of the health; the diagnosis of the medical or health condition; or the promotion or maintenance of the beauty.

The term "drug ion" as used in the specification of the present invention refers to an ion which is produced by the dissociation of a drug to ions and which plays a role in a drug effect or a pharmacological action.

The term "drug solution" as used in the specification of the present invention refers to a fluid substance containing the drug ion. The term "drug solution" as used in the specification of the present invention includes not only liquid states such as a solution prepared by dissolving a drug into a solvent and a stock solution when a drug is in a liquid state, but also various states such as a drug suspended or emulsified into a solvent or the like and the drug adjusted to an ointment shape or a paste shape as long as at least part of the drug dissociates to drug ions.

The term "drug counter ion" as used in the specification of the present invention refers to an ion which is present in the drug solution and which has a polarity opposite to a drug ion.

The term "skin" as used in the specification of the present invention refers to the surface of an organism to which a drug ion can be administered by iontophoresis, and includes a mucosa in an oral cavity. The term "organism" as used in the specification of the present invention refers to a human being or an animal.

The term "biological counter ion" as used in the specification of the present invention refers to an ion which is present on the skin of an organism or in the organism and which has a polarity opposite to a drug ion.

The term "front surface side" as used in the specification of the present invention refers to a side close to the skin of an organism on a current path flowing in the device upon administration of a drug ion.

Known examples of an ion exchange membrane include various ion exchange membranes such as: an ion exchange resin formed into a membrane shape; a heterogeneous ion exchange membrane obtained by dispersing an ion exchange resin into a binder polymer and by forming the resultant into a membrane through, for example, molding under heat; and a homogeneous ion exchange membrane obtained by impregnating and filling a base material such as a cloth, a net, or a porous film with a solution prepared by dissolving, into a solvent, a composition composed of a monomer, crosslinkable monomer, polymerization initiator, or the like in which an ion exchange group can be introduced, or a resin having a functional group in which an ion exchange group can be introduced, by subjecting the resultant to polymerization or solvent removal, and by subj ecting the resultant to a treatment for introducing an ion exchange group. The ion exchange membrane of the present invention can use any of those ion exchange membranes without particular limitation.

The cation exchange membrane in this description is an ion exchange membrane having a function of blocking the passage of an anion while permitting the passage of a cation (that is, an ion exchange membrane through which the cation passes more easily than the anion does). Specific examples of the membrane include Neosepta CM-1, Neosepta CM-2, Neosepta CMX, Neosepta CMS, and Neosepta CMB each manufactured by Tokuyama Corporation.

Similarly, the anion exchange membrane in this description is an ion exchange membrane having a function of blocking the passage of a cation while permitting the passage of an anion (that is, an ion exchange membrane through which the anion passes more easily than the cation does). Specific examples of the membrane include ion exchange membranes into which anion exchange groups are introduced such as Neosepta AM-1, Neosepta AM-3, Neosepta AMX, Neosepta AHA, Neosepta ACH, and Neosepta ACS each manufactured by Tokuyama Corporation.

As the ion exchange membrane of the present invention, an ion exchange membrane of a type in which a porous film is filled with an ion exchange resin can be particularly preferably used. Specifically, an ion exchange membrane obtained by filling a porous film with an ion exchange resin at a filling ratio of preferably 5 to 95 mass%, more preferably 10 to 90 mass%, or particularly preferably 20 to 60 mass% can be used, the porous film formed thereon having a large number of small pores having a mean pore size of preferably 0.005 to 5.0 µm, more preferably 0.01 to 2.0 µm, or most preferably 0.02 to 0.2 µm (a mean flow pore size measured according to the bubble point method (JIS K3832-1690)) at a porosity of preferably 20 to 95%, more preferably 30 to 90%, or most preferably 30 to 60% and having a film thickness of preferably 5 to 140 µm, more preferably 10 to 120 µm, or most preferably 15 to 55 µm.

The expression "blocking the passage of an ion" to be described for an ion selective membrane or an ion exchange membrane in this description does not necessarily mean that no ion is allowed to pass, and includes, for example, the case where a function requested of the ion selective membrane or the ion exchange membrane is sufficiently exerted because a rate at or an amount in which a specific ion passes through each of the membranes is sufficiently small as compared to that of another specific ion. Similarly, the expression "allowing the passage of an ion" to be described for an ion selective membrane or an ion exchange membrane does not mean that no restriction is imposed on the passage of the ion, and includes, for example, the case where a rate at or an amount in which the ion passes through each of the ion selective membrane and the ion exchange membrane is secured to such an extent that a function requested of each of the membranes is sufficiently exerted even when the passage of the ion is restricted to some extent.

### Brief Description of the Drawings

Fig. 1 is an explanatory view for showing the configuration of an iontophoresis device according to one embodiment of the present invention.
Fig. 2 is an explanatory view for showing the configuration of the iontophoresis device according to another embodiment of the present invention.
Figs. 3 (A) and 3 (B) are each an explanatory view for showing the configuration of the iontophoresis device according to another embodiment of the present invention.
Fig. 4 is an explanatory view for showing the configuration of a conventional iontophoresis device.
Fig. 5 is an explanatory view for showing the configuration of a working electrode assembly disclosed as one embodiment in the prior application.

### Best Mode for carrying out the Invention

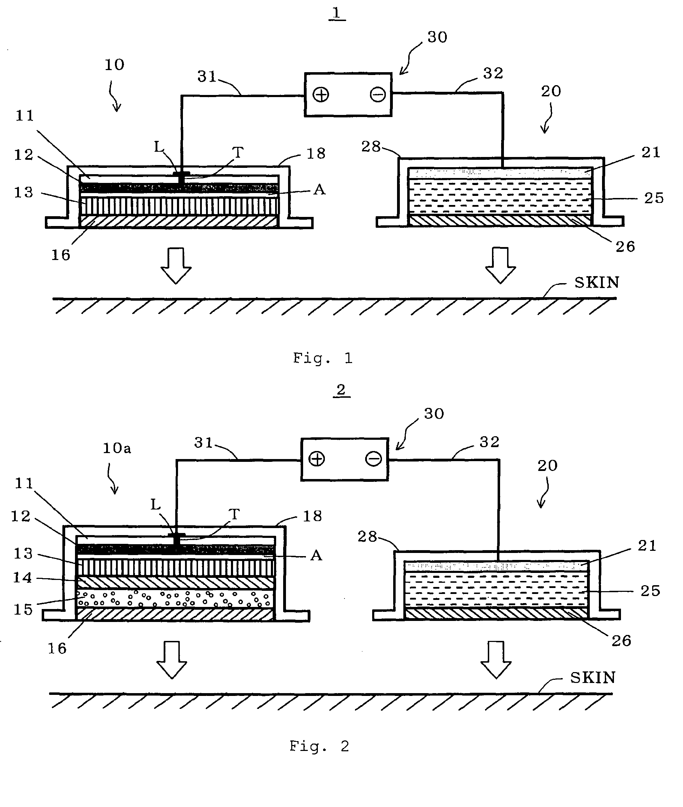

Fig. 1 is an explanatory view for showing the configuration of an iontophoresis device 1 according to one embodiment of the present invention.

As shown in figures, the iontophoresis device 1 is formed mainly of a working electrode assembly 10, a non-working electrode assembly 20, and an electric power source 30.

The working electrode assembly 10 includes a collector 12 connected to plus pole of the electric power source 30 through an electric supply line 31 and a polarizable electrode 13 placed on the front surface side of the collector 12, and its entirety is stored in a container 18.

On the other hand, the non-working electrode assembly 20 includes an electrode 21 connected to minus pole of the electric power source 30 through an electric supply line 32 and an electrolyte solution holding portion 25 for holding an electrolyte solution in contact with the electrode 21, and its entirety is stored in a container 28.

The above collector 12 is a member for supplying a current from the electric supply line 31 to as wide an area as possible of the polarizable electrode 13 at a uniform current density.

An arbitrary conductive material such as a metal can be used in the collector 12; a conductive material having a smaller specific resistivity or a smaller sheet resistivity than that of the electric conductor of which the polarizable electrode 13 is formed is preferably used. In addition, carbon fibers or carbon fiber paper can be particularly preferably used in the collector 12 for the purpose of, for example, preventing the elution of a metal component from the collector 12 in the case where a drug solution contacts the collector 12. In this case, a collector having a terminal member obtained by mixing a polymer matrix with carbon and a conductive sheet attached to the terminal member and composed of carbon fibers or carbon fiber paper, the collector being disclosed in Japanese Patent Application No. 2004-317317 by the applicant of the present application, or a collector having a conductive sheet portion composed of carbon fibers or carbon fiber paper, and an extension portion which is composed of carbon fibers or carbon fiber paper and part of which is impregnated with a water-repellent polymer, the collector being disclosed in Japanese Patent Application No. 2005-222892, can be particularly preferably used.

Alternatively, as shown in the figure, a manufacturing cost related to the formation of the collector 12 can be reduced by constituting the collector 12 from a conductive coating film formed by applying a conductive coating containing a conductive powder such as a carbon powder onto a substrate 11 made of, for example, plastic. In this case, the electric supply line 31 and the collector 12 can be electrically connected to each other by an arbitrary method; the figure shows an example in which a land L formed on the back surface of the substrate 11 and the collector 12 are connected to each other through a through-hole T formed in the substrate 11, and the electric supply line 31 is connected to the land L with, for example, a conductive adhesive.

A plate-or membrane-like member containing an electric conductor or activated carbon having a capacitance per unit volume of 100 mF/g or more or a specific surface area of 1 m²/g or more can be used in the polarizable electrode 13.

A particularly preferable configuration of the polarizable electrode 13 is, for example, as follows: a composition obtained by blending 97 to 80 parts by weight of an activated carbon powder having a specific surface area of about 100 m²/g with 3 to 20 parts by weight of a binder polymer such as polytetrafluoroethylene or polyvinylidene fluoride is molded into a membrane shape.

A more preferable configuration of the polarizable electrode 13 is, for example, a woven or non-woven fabric made of activated carbon fibers, or the woven or non-woven fabric impregnated with about 3 to 20 parts by weight of a binder polymer such as polytetrafluoroethylene or polyvinylidene fluoride. Activated carbon fibers obtained by carbonizing and activating novoloid fibers each of which: has an extremely high specific surface area (for example, 1,000 to 2,500 m²/g) and a high tensile strength (for example, 300 to 400 N/mm²); and is excellent in flexibility can be preferably used as the activated carbon fibers in this case. The activated carbon fibers obtained by carbonizing and activating novoloid fibers are available from, for example, Nippon Kynol Inc. under the trade name "Kynol activated carbon fibers".

The polarizable electrode 13 adsorbs a drug, and holds a drug solution composed of an aqueous solution in which a salt of a drug ion and an acid is dissolved by being impregnated with the drug solution.

The polarizable electrode 13 can be caused to adsorb the above drug, and can be impregnated with the drug solution by the following procedure.

That is, a treatment for causing the polarizable electrode 13 to adsorb the drug is performed as a first process. Here, the drug (second drug) to be adsorbed by the polarizable electrode 13 is a drug of the same kind as that of a drug (first drug) in the drug solution with which the polarizable electrode 13 is impregnated in a second process to be described later. The treatment can be performed by impregnating the polarizable electrode 13 with a solution prepared by dissolving the drug (second drug) in an organic solvent, and, preferably, by drying the resultant. A lower alcohol excellent in biological safety and having moderately high volatility such as ethanol can be preferably used as the organic solvent in this case. In addition, the polarizable electrode 13 can be impregnated with the above solution by dropping the above solution to the polarizable electrode 13, or by immersing the polarizable electrode 13 in the above solution.

Alternatively, the above first process of causing the polarizable electrode 13 to adsorb the drug ion can be performed by impregnating the polarizable electrode 13 with an aqueous solution in which a salt of the drug (second drug) and an acid is dissolved, preferably washing the resultant with water, and drying the resultant. The polarizable electrode 13 can be impregnated with the above aqueous solution by dropping the above aqueous solution to the polarizable electrode 13, or by immersing the polarizable electrode 13 in the above aqueous solution.

A treatment for impregnating the polarizable electrode 13 with the drug solution in which the salt of the drug (first drug) and the acid is dissolved is performed as a second process after the above first process. The treatment can be performed by, for example, dropping the drug solution to the polarizable electrode 13, or immersing the polarizable electrode 13 in the drug solution.

It should be noted that the polarizable electrode 13 is preferably treated with glycerin (a third process) prior to the above first process particularly when a polarizable electrode containing activated carbon fibers is used as the polarizable electrode 13.

That is, by reason of, for example, the fact that the activated carbon fibers are hydrophobic, even when the solution prepared by dissolving the second drug in the organic solvent or the aqueous solution in which the salt of the second drug ion and the acid is dissolved is brought into contact with the activated carbon fibers which are not treated, the solution or the aqueous solution does not easily permeate into the activated carbon fibers, but the permeability of the solution or the aqueous solution can be improved by treating the polarizable electrode 13 containing the activated carbon fibers with glycerin, whereby the first process can be performed in an additionally smooth fashion.

Blending the drug solution with which the polarizable electrode 13 is impregnated in the second process with a thickener to adjust the viscosity of the drug solution can improve the property with which the drug solution is held in the polarizable electrode 13. As a result, the ease of handling of the polarizable electrode 13 and the ease with which the device is assembled can be improved. A particularly preferable example of the thickener that can be used in this case is hydroxypropylcellulose (HPC). The content at which HPC is blended is appropriately in the range of about 1 to 5%.

In this embodiment, the polarizable electrode 13 is formed to hold the drug solution by being impregnated with the drug solution, so a polarizable electrode containing activated carbon or activated carbon fibers into which the drug solution can permeate excellently is particularly preferably used as the polarizable electrode 13.

The collector 12 and the polarizable electrode 13 can be bonded to each other with a conductive adhesive A in order that a state where the collector 12 and the polarizable electrode 13 are mechanically and/or electrically connected to each other may be favorably kept. A conductive adhesive using a carbon powder as a conductive filler is preferably used as the conductive adhesive A for the purpose of, for example, preventing the elution of a metal ion when the drug solution contacts the collector 12.

The working electrode assembly 10 can further include an ion selective membrane 16 on the front surface side of the polarizable electrode 13 as an arbitrary component.

A membrane-like member having such characteristic as to block the passage of a biological counter ion while permitting the passage of the drug ion in the polarizable electrode 13 is preferably used as the ion selective membrane 16. As a result, the efficiency with which the drug ion is administered can be improved.

The ion selective membrane 16 can adopt a semi-permeable membrane shape which permits and blocks the passage of the above ions on the basis of the molecular weights, sizes, or steric shapes of the ions. Alternatively, the membrane can adopt a charge selective membrane shape which permits and blocks the passage of the above ions on the basis of charge. A cation exchange membrane can be particularly preferably used as the ion selective membrane 16 of a charge selective membrane shape.

When a cation exchange membrane is used as the ion selective membrane 16, the polarizable electrode 13 and the cation exchange membrane 16 are preferably prevented from contacting each other by, for example, placing a separating member formed of an insulating material capable of permitting the passage of an ion between the polarizable electrode 13 and the cation exchange membrane 16 in order that the generation of a gas near the cation exchange membrane 16 which occurs when energization is performed by bringing the polarizable electrode 13 and the cation exchange membrane 16 into contact with each other may be prevented. The separating member in this case is preferably caused to hold a drug solution having the same composition as that of the drug solution with which the polarizable electrode 13 is impregnated. As a result, the property with which energization from the polarizable electrode 13 to the cation exchange membrane 16 occurs can be improved.

An arbitrary conductive material can be used in the electrode 21 without any particular limitation; the use of an electrode using a conductive material having a lower oxidation-reduction potential than that of water such as a silver-silver chloride electrode, or of a polarizable electrode can inhibit or suppress the generation of a gas or an unfavorable ion due to the electrolysis of water. When a polarizable electrode is used as the electrode 21, the property with which energization from the polarizable electrode to the electrolyte solution held by the electrolyte solution holding portion 25 occurs can be improved by impregnating the polarizable electrode with an electrolyte solution having composition identical to or different from that of the above electrolyte solution.

An inert electrode made of, for example, gold, platinum, stainless steel, or carbon can also be used as the electrode 21 in, for example, the case where the generation of a gas or an unfavorable ion due to the electrolysis of water can be prevented by, for example, appropriately selecting the electrolyte solution held by the electrolyte solution holding portion 25 or the case where an electrode reaction in the non-working electrode assembly 20 is no longer of concern by virtue of, for example, a small energization amount.

The electrolyte solution holding portion 25 can hold an electrolyte solution in which an arbitrary electrolyte capable of securing the property with which energization from the electrode 21 to the skin of an organism occurs is dissolved. Of such electrolyte solutions, a buffer electrolyte solution prepared by using an electrolyte having a lower oxidation-reduction potential than that of water or by dissolving multiple kinds of electrolytes is preferable because the generation of a gas or the production of an ion due to the electrolysis of water upon energization, or a pH change due to the generation or production can be suppressed.

An electrolyte solution capable of achieving the above object is, for example, a liquid prepared by mixing 0.5 M sodium fumarate and 0.5 M polyacrylic acid at a ratio of 5 _{:} 1.

The electrolyte solution holding portion 25 can hold the electrolyte solution in a liquid state, or can hold the solution by impregnating an appropriate absorbable carrier such as a woven or non-woven fabric made of natural or artificial fibers, a porous membrane, or gel with the solution.

An ion selective membrane 26 having such characteristic as to block the passage of a positive ion from the side of a living organism to the electrolyte solution holding portion 25 and to permit the passage of a negative ion from the electrolyte solution holding portion 25 to the side of the living organism can be placed as an arbitrary member on the front surface side of the electrolyte solution holding portion 25. As a result, the stability of a balance between the positive and negative ions at an interface between the skin of the living organism and the device can be improved.

The ion selective membrane 26 can adopt a charge selective membrane shape which permits and blocks the passage of the above ions on the basis of charge. Alternatively, the membrane can adopt a semi-permeable membrane shape which permits and blocks the passage of the above ions on the basis of the molecular weights, sizes, or steric shapes of the ions. An anion exchange membrane can be particularly preferably used as the ion selective membrane 26.

The container 18 for the working electrode assembly 10 and the container 28 for the non-working electrode assembly 20 are each a member formed of an arbitrary material such as plastic, the member having a space capable of storing the above-mentioned respective elements formed in itself and having an open lower surface. The containers 18 and 28 can each be preferably formed of a flexible material capable of: preventing the evaporation of moisture from its inside or the contamination of foreign matter from its outside; and tracking the movement of an organism or irregularities on the skin of the organism. A liner (not shown) which: is composed of an appropriate material capable of preventing the evaporation of moisture or the inclusion of foreign matter during the storage of the iontophoresis device 1; and is removed upon use of the device can be patched to the lower surface of each of the containers 18 and 28. Further, a pressure sensitive adhesive layer can also be provided for, for example, the outer periphery of the lower surface of each of the containers 18 and 28 for improving adhesiveness between the device and the skin of an organism.

A battery, a constant voltage device, a constant current device, a constant voltage/current device, or the like can be used as the electric power source 30. In embodiments of the present invention, it is preferable to use a constant current device whose current can be adjusted in the range of 0.01 to 1.0 mA/cm², or preferably 0.01 to 0.5 mA/cm², and which operates under safe voltage conditions, specifically at 50 V or less, or preferably 30 V or less.

In the iontophoresis device 1, the drug ion in the polarizable electrode 13 is administered to the living organism by applying, from the electric power source 30, a positive voltage to the collector 12 and a negative voltage to the electrode 21 in a state where the polarizable electrode 13 (the ion selective membrane 16 when the device includes the ion selective membrane 16) and the electrolyte solution holding portion 25 (the ion selective membrane 26 when the device includes the ion selective membrane 26) are brought into contact with the skin of the living organism.

Since energization to the drug solution is performed through the polarizable electrode 13 in the working electrode assembly 10 of the iontophoresis device 1, an electrode reaction upon energization can be inhibited or suppressed, so the generation of a gas such as an oxygen gas or a chlorine gas, or of a harmful ion such as a hydrogen ion or a hypochlorite ion, or the alteration of the drug ion due to a chemical reaction can be prevented, or at least reduced.

Further, in the iontophoresis device 1, the treatment for causing the polarizable electrode 13 to adsorb the drug is performed, so a reduction in pH value of the drug solution caused by bringing the drug solution into contact with the polarizable electrode 13 (in other words, by impregnating the electrode with the drug solution) can be suppressed. Therefore, the deterioration of the members of which the working electrode assembly 10 is formed such as the container 18 and the adhesive A due to the reduction in pH value of the drug solution can be inhibited or suppressed. Alternatively, the range of choices of the materials to be used in such members can be extended. Alternatively, a preventing or alleviating effect on surface roughening or inflammation at the skin of the living organism when the drug ion is administered to the living organism in a state where the pH value of the drug solution is reduced can be expected from the device.

Hereinafter, the results of the experiments conducted for validating an effect of the invention in this embodiment will be described.

### <Example 1>

Activated carbon fibers ACC507-15 manufactured by Nippon Kynol Inc. (activated carbon fiber content 100%, mass per unit area 120 g/m², thickness 0.5 mm, specific surface area 1,500 m²/g) cut into pieces each having a diameter of 17 mm and treated with glycerin in advance were used in the polarizable electrode 13, and the electrode was impregnated with 100 µl of a 10% solution of lidocaine in ethanol (solution prepared by dissolving 10 parts by weight of lidocaine hydrochloride in 90 parts by weight of ethanol) by dropping the solution. After that, the resultant was dried with air by using a forced air flow oven WFO-500 for 10 minutes at a temperature heated to 40°C, whereby the polarizable electrode 13 was caused to adsorb lidocaine as the second drug (first process).

80 µl of a drug solution composed of a 10% aqueous solution of lidocaine hydrochloride (aqueous solution prepared by dissolving 10 parts by weight of lidocaine hydrochloride in 90 parts by weight of water) were dropped to the polarizable electrode 13 caused to adsorb lidocaine in the first process, whereby the polarizable electrode 13 was impregnated with the drug solution (second process).

### <Example 2>

The same polarizable electrode 13 as that of Example 1 was impregnated with 100 µl of a 5% solution of lidocaine in ethanol in the same manner as in Example 1. After that, the resultant was dried with air under the same conditions as those of Example 1, whereby the polarizable electrode 13 was caused to adsorb lidocaine as the second drug (first process).

A drug solution having the same composition as that of Example 1 was dropped in the same amount as that of Example 1 to the polarizable electrode 13 caused to adsorb lidocaine in the first process, whereby the polarizable electrode 13 was impregnated with the drug solution (second process).

### <Example 3>

The same polarizable electrode 13 as that of Example 1 was impregnated with 100 µl of a 10% aqueous solution of lidocaine (aqueous solution prepared by dissolving 10 parts by weight of lidocaine hydrochloride in 90 parts by weight of water) in the same manner as in Example 1. After that, the resultant was sufficiently washed under running water, and was then dried with air under the same conditions as those of Example 1, whereby the polarizable electrode 13 was caused to adsorb lidocaine as the second drug (first process).

A drug solution having the same composition as that of Example 1 was dropped in the same amount as that of Example 1 to the polarizable electrode 13 caused to adsorb lidocaine in the first process, whereby the polarizable electrode 13 was impregnated with the drug solution (second process).

### <Comparative Example>

The same polarizable electrode 13 as that of Example 1 was used. A drug solution having the same composition as that of Example 1 was dropped in the same amount as that of Example 1 to the polarizable electrode 13 while the treatment for causing the electrode to adsorb lidocaine as the second drug (first process) was not performed, whereby the polarizable electrode 13 was impregnated with the drug solution (second process).

Table 1 shows the results of the measurement of a change in pH value of the drug solution with which the polarizable electrode 13 was impregnated by the second process of each of Examples 1 to 3 and Comparative Example with time from the time point of the completion of the second process.

**[Table 1]**

| Elapsed time | 0 minutes | 1 hour | 1 day | 14 days |
|---|---|---|---|---|
| Example 1 | 5.7 | 5.2 | 4.7 | 4.4 |
| Example 2 | 5.7 | 4.8 | 4.2 | 4.0 |
| Example 3 | 5.6 | 5.2 | 4.6 | 4.3 |
| Comparative Example | 5.7 | 3.5 | 2.6 | 2.5 |

As is apparent from Table 1, the rate at which the pH value of the drug solution reduces in each of Examples 1 to 3 in each of which the treatment for causing the polarizable electrode 13 to adsorb the second drug was performed is clearly smaller than that in the case of Comparative Example in which the treatment for causing the polarizable electrode 13 to adsorb the second drug was not performed. In addition, in each of Examples 1 to 3, the following fact can be observed: the rate at which the pH value reduces tends to become smaller with a lapse of time from the completion of the second process, and the drug solution with which the polarizable electrode is impregnated maintains a pH value of about 4 to 4.5 even after a lapse of 14 days from the completion of the second process.

Therefore, in the iontophoresis device 1, even when the drug ion is administered after a lapse of a certain time period from the assembly of the device, a reduction in pH value of the drug solution during the time period can be significantly suppressed.

Fig. 2 is an explanatory view for showing the configuration of an iontophoresis device 2 according to another embodiment of the present invention.

The iontophoresis device 2 has the same configuration as that of the iontophoresis device 1 except that a working electrode assembly 10a of the device 2 includes not only the collector 12 and the polarizable electrode 13 identical to those of the working electrode assembly 10 but also a drug solution holding portion 15 on the front surface side of the polarizable electrode 13.

The drug solution holding portion 15 holds a drug solution in which a salt of a drug and an acid is dissolved. The drug solution held by the drug solution holding portion 15 can be a drug solution having the same composition as that of the drug solution with which the polarizable electrode 13 is impregnated.

The drug solution holding portion 15 can hold the drug solution in a liquid state, or can hold the solution by impregnating an appropriate absorbable carrier such as a woven or non-woven fabric made of natural or artificial fibers, a porous membrane, or gel with the solution.

The iontophoresis device 2 achieves an effect similar to that described above for the iontophoresis device 1 because the treatment for causing the polarizable electrode 13 to adsorb the drug (first process) is performed.

Further, the iontophoresis device 2 achieves an additional effect, that is, an increase in degree of freedom in the adjustment of the amount of the drug ion (or the drug solution) held in the working electrode assembly 10a because the device includes the drug solution holing portion 15.

In the iontophoresis device 2, the treatment for impregnating the polarizable electrode 13 with the drug solution (second process) described above for the iontophoresis device 1 can be omitted.

In that case, whether or not the drug solution in the drug solution holding portion 15 permeates into the polarizable electrode 13 by placing the drug solution holding portion 15 on the front surface side of the polarizable electrode 13 depends on a material of which the polarizable electrode 13 is formed; irrespective of whether or not the drug solution of the drug solution holding portion 15 permeates into the polarizable electrode 13, the drug solution at least contacts the polarizable electrode 13 at an interface between the polarizable electrode 13 and the drug solution holding portion 15.

However, in the iontophoresis device 2, a problem, specifically, an abrupt reduction in pH value of the drug solution in the drug solution holding portion 15 can be dissolved or alleviated because the treatment for causing the polarizable electrode 13 to adsorb the drug (first process) is performed before the process for placing the drug solution holding portion 15 on the front surface side of the polarizable electrode 13 is performed.

The iontophoresis device 2 can include an ion selective membrane 14 as an arbitrary component between the polarizable electrode 13 and the drug solution holding portion 15.

The ion selective membrane 14 preferably has such characteristic as to permit the passage of an anion from the drug solution holding portion 15 to the polarizable electrode 13 while blocking the passage of a cation from the polarizable electrode 13 to the drug solution holding portion 15. As a result, even when a harmful ion such as a hydrogen ion or a hypochlorite ion is generated at the polarizable electrode 13, the migration of such ion to an interface between the living organism and the device can be suppressed.

The ion selective membrane 14 can adopt a semi-permeable membrane shape which permits and blocks the passage of the above ions on the basis of the molecular weights, sizes, or steric shapes of the ions. Alternatively, the membrane can adopt a charge selective membrane shape which permits and blocks the passage of the above ions on the basis of charge. An anion exchange membrane can be particularly preferably used as the ion selective membrane 14 of a charge selective membrane shape.

It should be noted that, when an anion exchange membrane is used as the ion selective membrane 14, the polarizable electrode 13 and the anion exchange membrane 14 are preferably prevented from contacting each other by, for example, placing a separating member formed of an insulating material capable of permitting the passage of an ion between the polarizable electrode 13 and the anion exchange membrane 14 in order that the generation of a gas near the anion exchange membrane 14 which occurs when energization is performed by bringing the polarizable electrode 13 and the anion exchange membrane 14 into contact with each other may be prevented. The separating member in this case is preferably impregnated with a drug solution having the same composition as that of the drug solution with which the polarizable electrode 13 is impregnated. As a result, the property with which energization from the polarizable electrode 13 to the anion exchange membrane 14 occurs can be made favorable.

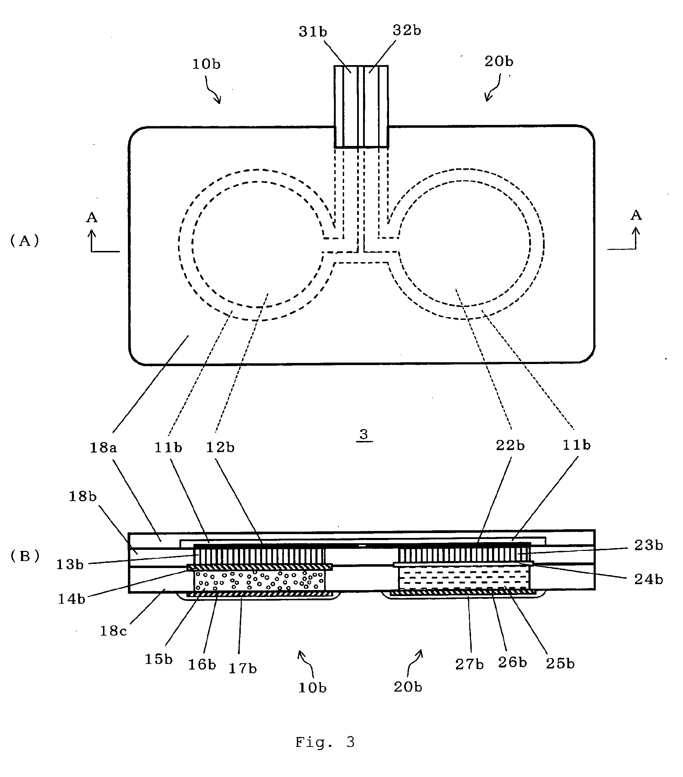

Fig. 3(A) is a plan view for showing an iontophoresis device 3 according to still another embodiment of the present invention, and Fig. 3(B) is a sectional view taken along the line A-A of Fig. 3(A).

The iontophoresis device 3 is formed of substantially the same elements as those of the iontophoresis device 2. In addition to the forgoing, an improvement in ease with which the iontophoresis device 3 is assembled and an improvement in ease of handling of the device are realized by appropriately selecting, for example, the material, dimensions, and shape of each member.

That is, the iontophoresis device 3 includes a working electrode assembly portion 10b composed of a substrate 11b, a collector 12b, a polarizable electrode 13b, and a drug solution holding portion 15b similar to the substrate 11, the collector 12, the polarizable electrode 13, and the drug solution holding portion 15 in the working electrode assembly 10a, and uses the polarizable electrode 13b, which has adsorbed a drug, as in the case of the iontophoresis device 2, so the device achieves an effect similar to that of the iontophoresis device 2 such as the suppression of a reduction in pH value of a drug solution contacting the polarizable electrode 13b.

A non-working electrode assembly portion 20b of the iontophoresis device 3 is formed of: an electrode composed of the substrate 11b, a collector 22b, and a polarizable electrode 23b; and an electrolyte solution holding portion 25b placed on the front surface side of the electrode. The collector 22b can have the same configuration as that of the collector 12b, and the polarizable electrode 23b can be formed of the same material as that of the polarizable electrode 13b. The property with which energization from the polarizable electrode 23b to the electrolyte solution of the electrolyte solution holding portion 25b occurs can be improved by impregnating the polarizable electrode 23b with an electrolyte solution having composition identical to or different from that of the above electrolyte solution.

The iontophoresis device 3 has a cover 18a, and supports 18b and 18c in each of which two openings each having a predetermined shape (such as a circular shape) are formed. The polarizable electrodes 13b and 23b are loaded into the openings of the support 18b, and the drug solution holding portion 15b and the electrolyte solution holding portion 25b are loaded into the openings of the support 18c. The substrate 11b having the collectors 12b and 22b formed on its front surface side is interposed between the cover 18a and the support 18b.

Therefore, in the iontophoresis device 3, the working electrode assembly portion 10b and the non-working electrode assembly portion 20b can be simultaneously assembled by a simple approach, that is, the overlapping and pasting of the following four sheet-like members:
(1) the cover 18a;
(2) the substrate 11b in which the collectors 12b and 22b are formed;
(3) the support 18b loaded with the polarizable electrodes 13b and 23b; and
(4) the support 18c loaded with the drug solution holding portion 15b and the electrolyte solution holding portion 25b.
As a result, in the iontophoresis device 3, for example, the following effects can be achieved: an improvement in ease with which the device is produced, a reduction in cost for the production of the device, and an increase in yield in which the device is produced.

In addition, as shown in the figures, the iontophoresis device 3 can be easily reduced in thickness, and the use of a flexible material such as foamable polyurethane in each of the cover 18a, the supports 18b and 18c, and the like can improve the property with which the device tracks irregularities on the skin of an organism or the movement of the organism.

It should be noted that the four sheet-like members in the foregoing can be pasted together with, for example, an adhesive layer formed on the front surface side of each of the cover 18a and the support 18b. However, the cover 18b can be omitted when the substrate 11b and the support 18b are bonded to each other with an adhesive layer formed on the front surface side of the substrate 11b or on the upper surface side of the support 18b. Alternatively, the supports 18b and 18c can be formed as a single member. As a result, additional simplification of the manufacture of the device, an additional reduction of a manufacturing cost for the device, and the like can be achieved.

The iontophoresis device 3 can include an ion selective membrane 14b, 16b, or 26b similar to the ion selective membrane 14, 16, or 26 in the iontophoresis device 1 or 2 as an arbitrary component. As a result, the device achieves an effect similar to that described above for the iontophoresis device 1 or 2. In this case, a spacer 24b for preventing the generation of a large step difference between the non-working electrode assembly portion 20b and the working electrode assembly portion 10b can be placed in the non-working electrode assembly portion 20b.

Further, the iontophoresis device 3 can include a layer 17b of a drug solution the viscosity of which is adjusted with a thickener such as HPC on the front surface side of the drug solution holding portion 15b, and a layer 27b of an electrolyte solution the viscosity of which is adjusted with a thickener such as HPC on the front surface side of the electrolyte solution holding portion 25b. As a result, an improvement in efficiency with which a drug ion is administered to a living organism by virtue of an improvement in adhesiveness between the skin of the living organism and the device can be achieved. Those layers 17b and 27b can each be an appropriate absorbable carrier such as a woven or non-woven fabric made of natural or artificial fibers, a porous membrane, or gel impregnated with the drug solution or the electrolyte solution.

In the iontophoresis device 3, a pressure-sensitive adhesive layer for improving adhesiveness between the device and the skin of the living organism can be formed on the front surface side of the support 18c. A peelable liner for preventing the inclusion of foreign matter or the drying of the drug solution and the electrolyte solution during the storage of the device can be further stuck to the front surface side of the layer.

Electric supply in the iontophoresis device 3 can be performed by connecting an unshown electric power source to the tips of electric supply lines 31b and 32b extended from the collectors 12b and 22b. The collectors 12b and 22b, and the electric supply lines 31b and 32b can be formed through the application of the same conductive coating by an approach such as screen printing.

The present invention has been described above on the basis of several embodiments. However, the present invention is not limited to those embodiments, and various modifications can be made to the present invention without departing from the description in the scope of claims of the present invention.

For example, in each embodiment described above, the case where a non-working electrode assembly includes an electrolyte solution holding portion has been described. However, the following procedure can also be adopted: the electrolyte solution holding portion is omitted, and the electrode of the non-working electrode assembly is directly brought into contact with the skin of an organism so that a drug ion is administered to the organism. Alternatively, the following procedure can also be adopted: an iontophoresis device itself is not provided with any non-working electrode assembly, and a voltage is applied to a working electrode assembly in accordance with the present invention in, for example, a state where the working electrode assembly is brought into contact with the skin of an organism and part of the organism is brought into contact with a member serving as an earth so that a drug is administered to the organism.

Although description has been given by taking an iontophoresis device including a single working electrode assembly and a single non-working electrode assembly as an example in each of the above embodiments, the present invention is applicable also to an iontophoresis device in which each of both two electrode assemblies connected to both terminals of an electric power source holds a drug to be administered to a living organism or to an iontophoresis device in which at least one pole of an electric power source has multiple working electrode assemblies and/or multiple non-working electrode assemblies connected to itself. In this case, as long as at least one electrode assembly holding a drug solution in which a salt of a drug and an acid is dissolved includes a polarizable electrode caused to adsorb a drug of the same kind as that of the above drug, the iontophoresis device is included in the scope of the present invention.

Although description has been given by taking the case where a collector for uniformly supplying electricity to a polarizable electrode is used as an example in each of the above embodiments, electric supply can be performed by directly connecting an electric supply line to the polarizable electrode without the use of the collector in, for example, the case where the specific resistivity or sheet resistivity of the polarizable electrode is sufficiently small.

The shape, dimensions, material, and the like of each member in each of the above embodiments are described merely as examples, and the present invention is not limited by the description.

## Claims

1. An iontophoresis device, **characterized in that** the iontophoresis device comprises an electrode assembly holding a drug solution into which a salt of a first drug and an acid is dissolved,
and **in that** the electrode assembly has a polarizable electrode to which a second drug of the same kind as that of the first drug is adsorbed, the polarizable electrode contacting with the drug solution.

2. The iontophoresis device according to claim 1, **characterized in that** the adsorption of the second drug to the polarizable electrode is caused by treating the polarizable electrode with a solution prepared by dissolving the second drug into an organic solvent.

3. The iontophoresis device according to claim 1, **characterized in that** the adsorption of the second drug to the polarizable electrode is caused by treating the polarizable electrode with an aqueous solution into which a salt of the second drug and an acid is dissolved.

4. The iontophoresis device according to any one of claims 1 to 3, **characterized in that** the adsorption of the second drug is performed on the polarizable electrode that has been treated with glycerin in advance.

5. The iontophoresis device according to any one of claims 1 to 4, **characterized in that** the polarizable electrode is impregnated with the drug solution.

6. The iontophoresis device according to any one of claims 1 to 5, **characterized in that** a drug solution holding portion holding the drug solution is placed on a front surface side of the polarizable electrode.

7. The iontophoresis device according to any one of claims 1 to 6, **characterized in that** the polarizable electrode comprises an electrode containing activated carbon.

8. The iontophoresis device according to any one of claims 1 to 7, **characterized in that:**
the first drug comprises lidocaine; and
the salt of the first drug and the acid comprises lidocaine hydrochloride.

9. A method of producing an iontophoresis device including an electrode assembly holding a drug solution into which a salt of a first drug and an acid is dissolved, the electrode assembly having a polarizable electrode contacting with the drug solution, the method being **characterized by** comprising:
a first process of adsorbing a second drug of the same kind as that of the first drug to the polarizable electrode; and
a second process of contacting the drug solution with the polarizable electrode, the second process being performed after the first process.

10. The method of producing an iontophoresis device according to claim 9, **characterized in that** the first process includes
a first step of impregnating the polarizable electrode with a solution prepared by dissolving the second drug into an organic solvent, and
a second step of drying the polarizable electrode to vaporize the organic solvent.

11. The method of producing an iontophoresis device according to claim 9, **characterized in that** the first process includes:
a first step of impregnating the polarizable electrode with an aqueous solution into which a salt of the second drug and an acid is dissolved;
a second step of washing the polarizable electrode with water, the second step being performed after the first step; and
a third step of drying the polarizable electrode to vaporize moisture, the third step being performed after the second step.

12. The iontophoresis device according to any one of claims 9 to 11, **characterized in that:**
the method further comprises a third process of treating the polarizable electrode with glycerin; and
the first process is performed after the third process.
